(19) Europäisches Patentamt
European Patent Office
Office européen des brevets

(11) Numéro de publication: **0 254 616 A1**

(12) # DEMANDE DE BREVET EUROPEEN

(21) Numéro de dépôt: 87401509.2

(22) Date de dépôt: 30.06.87

(51) Int. Cl.[3]: C 07 K 15/00 //A61K35/36, A61K35/50, A61K37/02

**Une requête en rectification page 13 de la description a été présentée conformément à la règle 88 CBE. Il est statué sur cette requête au cours de la procédure engagée devant la division d'examen.**

(30) Priorité: 30.06.86 FR 8609476

(43) Date de publication de la demande: 27.01.88 Bulletin 88/4

(84) Etats contractants désignés: AT BE CH DE ES FR GB GR IT LI LU NL SE

(71) Demandeur: CENTRE NATIONAL DE LA RECHERCHE SCIENTIFIQUE (CNRS)
13 Quai Anatole France
F-75700 Paris(FR)

(72) Inventeur: Courtois, Yves Germain Constant
59 Rue Thiers
F-92100 Boulogne(FR)

(72) Inventeur: Gulino née Debrac, Danielle
4 Allée des Jonquilles
F-93360 Neuilly-Plaisance(FR)

(72) Inventeur: Jozefonvicz née Dorgebray, Jacqueline
65 Deuxième Avenue Lelys
F-60260 Lamorlaye(FR)

(72) Inventeur: Jozefowicz, Marcel
65 Deuxième Avenue Lelys
F-60260 Lamorlaye(FR)

(72) Inventeur: Uhlrich née Noureux, Sylvie Madeleine
201-203 rue d'Alésia
F-75014 Paris(FR)

L'autre ióventeur a renonce a sa designation

(74) Mandataire: Phélip, Bruno et al,
c/o Cabinet Harlé & Phélip 21, rue de la Rochefoucauld
F-75009 Paris(FR)

(54) **Procédé d'obtention de nouvelles fractions protéiques à partir des activités facteurs de croissance, ces fractions et leur application comme agents de stimulation et de potentialisation des activités facteurs de croissance.**

(57) On purifie un facteur de croissance, par chromatographie d'affinité sur une résine I à base de polysaccharides sur lesquels est fixée de l'héparine, afin d'obtenir une fraction F constituée par des traces du facteur aFGF et d'une nouvelle fraction protéique, en réalisant une élution avec un tampon pH neutre, d'une force ionique équivalente à celle d'une solution de NaCl voisine de 1M, et on purifie la fraction F précitée, par chromatographie d'affinité sur une résine II, en réalisant une élution avec un tampon pH neutre, d'une force ionique équivalente à celle d'une solution de NaCl voisine de 2M, la résine II étant un dextrane réticulé présentant des fonctions carboxyméthyle, éventuellement carboxyméthylbenzylamide, et carboxyméthylbenzylamide sulfonate, et dont le comportement biologique est celui de l'héparine. La nouvelle fraction protéique est caractérisée par le fait que son poids moléculaire est déterminé comme étant de 20 000 D en électrophorèse en condition non réductrice (spectre à bande unique) ; et comme étant de 19 000 D en électrophorèse en condition réductrice (spectre à deux bandes), et que, par HPLC, elle fournit un spectre à trois pics.

EP 0 254 616 A1

La présente invention se rapporte à un procédé pour l'obtention de nouvelles fractions protéiques, à partir des activités facteurs de croissance ; elle se rapporte également aux fractions protéiques ainsi obtenues, ainsi qu'à leur application comme agents de stimulation et de potentialisation des activités facteurs de croissance. En effet, ces nouvelles fractions protéiques présentent une activité au moins partielle de co-facteur des facteurs de croissance. La présente invention concerne donc de façon générale l'isolement de telles activités co-facteurs.

Dans la littérature, on a décrit des activités facteurs de croissance qui sont obtenues à partir d'un grand nombre de tissus, notamment à partir de l'hypophyse, du cerveau, de l'hypothalamus, de la rétine et de différents tissus oculaires, du cartilage et des chondrosarcomes, et également du rein, du foie, du placenta, du corpus lutéum, des muscles, etc.

Il ressort de nombreux travaux de recherches que la plupart de ces facteurs appartiennent à deux groupes ayant ces activités.

Ces protéines ont été séparées et caractérisées par différents procédés de purification, et leur structure primaire a été élucidée.

Le premier groupe de ces protéines a été décrit sous les noms de FGF (ou basic FGF), AGF2 (astroglial growth factor), EDGFI, βHBGF (bêta heparin binding growth factor) ; quant au deuxième, il a été décrit sous les noms de ECGF, acidic FGF, AGF1, EDGFII, RDGF (retinal derived growth factor), α-HBGF. Par simplification, ces facteurs de croissance connus seront dénommés bFGF et aFGF. A titre de références bibliographiques concernant les facteurs de

croissance, on citera Roy R. Lobb et al dans "Analytical Biochemistry 154, 1-14 (1986) et J. Courty et al dans "Biochem. Biophys. Res. Commun., 136, 102-108 (1986)".

Dans la présente description, l'expression "activités facteurs de croissance" désigne des extraits contenant des composés capables d'agir sur la prolifération cellulaire. Les références bibliographiques sus-mentionnées décrivent également les méthodes de mise en évidence de l'activité biologique de ces facteurs de croissance.

Il a maintenant été découvert qu'en réalisant des chromatographies, sur des résines particulières, de ces activités facteurs de croissance, on pouvait isoler des fractions protéiques distinctes des facteurs de croissance, ces fractions protéiques présentant la propriété intéressante de stimuler l'activité biologique de ces facteurs de croissance.

La présente invention a donc d'abord pour objet un procédé pour l'obtention d'une fraction protéique ayant une activité au moins partielle de co-facteur des facteurs de croissance, caractérisé par le fait qu'il consiste à purifier un facteur de croissance par chromatographie d'affinité sur une résine I et/ou II,

- ladite résine I étant à base de polysaccharides sur lesquels est fixée de l'héparine, et
- la résine II étant constituée d'un dextrane réticulé qui présente des fonctions carboxyméthyle, éventuellement des fonctions carboxyméthylbenzylamide et qui présente également des fonctions carboxyméthylbenzylamide sulfonate, et dont le comportement biologique est celui de l'héparine,

en réalisant, dans le cas de la chromatographie d'affinité sur la résine I, une élution avec un tampon pH neutre, d'une force ionique équivalente à celle d'une solution de NaCl voisine de 1 M, et, dans le cas de la chromatographie d'affinité sur la résine II, une élution avec un tampon pH

neutre, d'une force ionique equivalente à celle d'une solution de NaCl voisine de 2 M.

Conformément a un premier mode de réalisation de la présente invention, on purifie le facteur de croissance par chromatographie d'affinité sur la résine I, afin d'obtenir une fraction F, constituée par des traces du facteur aFGF et par une fraction protéique possédant la propriété de stimuler et potentialiser les facteurs de croissance, puis on purifie ladite fraction F par chromatographie d'affinité sur la résine II, afin d'obtenir la fraction protéique précitée possédant la propriété de stimuler et potentialiser les facteurs de croissance. Cette fraction protéique est particulièrement intéressante ; elle est décrite en tant que telle ci-après.

Conformément à un deuxième mode de réalisation de la présente invention, on purifie le facteur de croissance par chromatographie d'affinité sur la résine I seulement pour obtenir ladite fraction F. Dans le cas du procédé conforme à ce second mode de réalisation, on obtient donc une fraction protéique moins pure que celle que l'on obtient en mettant en oeuvre le procédé conforme au premier mode de réalisation, mais qui possède tout de même l'activité co-facteur des facteurs de croissance.

Conformément à un troisième mode de réalisation du procédé selon l'invention, on purifie le facteur de croissance par chromatographie d'affinité sur la résine II. L'intérêt du procédé conforme ce troisième mode de réalisation est que les facteurs de croissance aFGF et bFGF sont faiblement retenus sur la résine II, la fraction protéique intéressante s'adsorbe sur la colonne de chromatographie à 0,65M NaCl (ou une force ionique équivalente) et est éluée à 2M NaCl (ou une force ionique équivalente) ; il en résulte une meilleure séparation de la fraction intéressante recherchée.

On utilise, comme matériel de départ, un facteur de croissance d'origine humaine ou animale. En particulier,

on peut mentionner un facteur de croissance issu du cerveau humain ou du placenta humain. On peut également partir du cerveau bovin.

Conformément à un mode de réalisation préféré du procédé de l'invention, on utilise, comme matériel de départ, un extrait acido-soluble de cerveau humain ou bien un extrait placentaire humain.

L'extrait acido-soluble de cerveau humain peut être obtenu par la succession d'étapes suivantes :

- broyage du cerveau humain en tampon phosphate à pH neutre;
- obtention des protéines par précipitation au sulfate d'ammonium entre 20 et 60% ;
- isolement des protéines restant solubles en milieu acide, au voisinage de pH 3 ; et
- dialyse contre un tampon à pH neutre.

Comme indiqué ci-dessus, la résine I est à base de polysaccharides sur lequel est fixée de l'héparine. (L'héparine est un mélange de glycosaminoglycanes extraits de l'intestin de porc et présentant une large distribution de poids moléculaires ; l'héparine purifiée utilisée est majoritairement constituée d'une séquence linéaire de disaccharide d'acide α-L-idopyrannuronique-2-sulfate et de 2-déoxy-2-sulfamino-α-D-glucopyrannose-6-sulfate). Les meilleurs résultats ont été obtenus avec une résine I commercialisée sous la dénomination "Heparin Sépharose" par la Société "Pharmacia".

Dans la chromatographie d'affinité avec la résine II, les meilleurs résultats ont été obtenus avec une résine II du type de celle décrite dans la publication de H. Mauzac, N. Aubert et J. Jozefonvicz : "Antithrombic activity of some polysaccharides resins", Biomaterials, 1982, 3, 221-224, en particulier avec la résine "Séphadex carboxyméthylbenzylamide sulfonate", qui possède une activité "heparin-like" de 0,2 UT/mg et dont la structure est un polymère constitué par les motifs suivants :

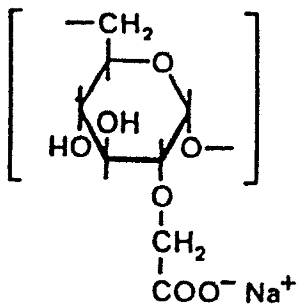

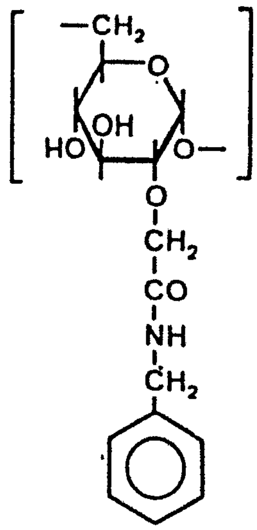

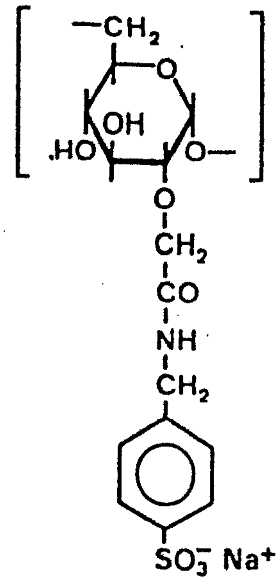

$[COO^-] = 81\ \%$

$[SO_3^-] = 9\ \%$

On peut également séparer en sous-fractions la fraction protéique possédant l'activité de co-facteur par la technique de HPLC, lesdites sous-fractions ayant également cette activité.

La fraction protéique, obtenue notamment par le procédé selon le premier mode de réalisation, est caractérisée en elle-même par le fait que son poids moléculaire est déterminé comme étant de 20 000 daltons environ en électrophorèse en condition non réductrice, laquelle fournit un spectre à bande unique, et comme étant de 19 000 daltons environ en électrophorèse en condition réductrice, laquelle fournit un spectre à deux bandes, et que, par la méthode de chromatographie en phase liquide haute performance, elle fournit un spectre à trois pics.

Sa composition en acides aminés, déterminés après hydrolyse acide et avant HPLC phase inverse, est donnée dans le tableau suivant :

TABLEAU

Composition en acides aminés de cette fraction protéique (calculée par rapport à un poids moléculaire de 19 000 daltons)

| Acide aminé | Nombre de résidus par molécules |
|---|---|
| Asx | 12 |
| Glx | 18 |
| Ser | 15 |
| His | 5 |
| Gly | 23 |
| Thr | 11 |
| Arg | 15 |
| Ala | 14 |
| Tyr | 4 |
| Met | 3 |
| Val | 6 |
| Phe | 6 |
| Ile | 4 |
| Leu | 9 |
| Lys | 26 |

Cette composition indique que la fraction protéique est différente des facteurs de croissance aFGF et bFGF et qu'elle est caractérisée par la présence d'un nombre important de résidus lysine.

En outre, comme indiqué ci-dessus, cette fraction protéique peut également être caractérisée par le fait qu'elle possède la propriété de stimuler et potentialiser les facteurs de croissance.

La présente invention porte également sur chacune des trois sous-fractions résultant d'une séparation par la technique de HPLC de la fraction protéique qui a été définie ci-dessus.

Les applications des fractions protéiques selon l'invention, en particulier de la fraction F protéique décrite ci-dessus ainsi que des trois sous-fractions précitées, sont toutes celles dans lesquelles sont impliqués les facteurs de croissance : pour la fabrication de milieux de culture ; en cosmétologie ; pour la cicatrisation de la peau et de la cornée; dans des applications thérapeutiques, par exemple pour la potentialisation de l'effet angiogénique des facteurs de croissance.

Dans ce qui suit, on décrit l'obtention de la fraction protéique selon l'invention à partir d'un extrait acido-soluble d'un facteur de croissance issu du cerveau humain, conformément aux deux premiers procédés mentionnés ci-dessus, ainsi que la mise en évidence de son activité stimulatrice et potentialisatrice de l'activité biologique des facteurs de croissance. On a également décrit l'obtention de la fraction protéique à partir d'un extrait placentaire comportant un facteur de croissance.

## I - OBTENTION DE LA FRACTION PROTEIQUE SELON L'INVENTION A PARTIR D'UN EXTRAIT ACIDO-SOLUBLE DE CERVEAU

### Ia - MISE EN OEUVRE DU PREMIER PROCEDE

(a) Préparation de l'extrait acido-soluble de cerveau.

Toutes les opérations ont lieu à 4°C. 4 kg de tissus sont homogénisés à l'aide d'un broyeur Turax dans 1 l de tampon PBS. Les débris cellulaires sont éliminés par centrifugation à 10 000g pendant 45 mn (Rotor Beckmann JA 10). Le surnageant, appelé extrait brut, est ajusté à une concentration de 20% $(NH_4)_2SO_4$ et centrifugé 45 mn à

10 000 g. Le deuxième surnageant est passé sur laine de verre, afin d'éliminer les traces de lipides, puis ajusté à une concentration de 60% $(NH_4)_2SO_4$ et centrifugé à 10 000 g pendant 45 mn. Le précipité obtenu est redissous dans un volume minimum de PBS (environ 400 ml) et la solution est ajustée à pH 3,2 avec de l'acide acétique glacial, puis immédiatement centrifugée pendant 30mn à 10 000 g. Le surnageant est réajusté à pH 7 avec de la soude concentrée et dialysé extensivement contre du PBS. La solution est ensuite centrifugée 30 mn à 20 000 g (Rotor Beckmann JA 20), afin d'éliminer toute trace de précipité. Ce dernier surnageant est appelé extrait acido-soluble.

(b) Chromatographie d'affinité sur la résine I (Heparin Sepharose)

4 g de résine ( ou gel) Heparin-Sepharose sont mis à gonfler pendant 15 mn dans 100 ml de tampon PBS et coulés en colonne de 1,6 x10 cm (16 ml) à 12,5 ml/h/$cm^2$. 120 mg de l'extrait acido-soluble de cerveau obtenu à l'étape (a) par g de gel son déposés sur la colonne. Les protéines retenues sont éluées successivement avec des solutions de PBS ajustées respectivement à 0,65M, 1M et 2M NaCl. Des fractions de 5ml sont collectées et dosées en quantité de protéines et en activité biologique.

La figure 1 du dessin annexé représente le chromatogramme obtenu. On constate l'existence :

- d'une fraction de tête F (1,10), éluée à 1M NaCl, suivie
- d'une fraction $F_1$ (11,25), encore éluée à 1M NaCl; et
- d'une fraction $F_2$ (30,40), éluée à 2M NaCl.

La figure 2 représente le spectre de ces trois fractions F, $F_1$ et $F_2$, lequel a été obtenu par électrophorèse en condition réductrice sur gel de polyacrylamide . L'observation de ce spectre montre que la fraction F (ligne a) est constituée par une nouvelle fraction protéique, avec, à l'état de traces, le facteur h-aFGF, la fraction $F_1$ correspondant au facteur h-aFGF (ligne b) et la

fraction $F_2$ ,au facteur h-bFGF (ligne c) .

(c) Chromatographie d'affinité sur la résine II (résine "Sephadex carboxyméthylbenzylamide sulfonate")

La fraction F, recueillie à l'étape précédente, est ensuite soumise à une chromatographie d'affinité sur la résine II sus-indiquée. Le mode opératoire est le suivant :

Une colonne 2,8 x 16 cm (100 ml) est équilibrée avec du PBS à un débit de 10 ml/h/$cm^2$ : 1 mg de protéines par millilitre de résine est déposé sur le haut de la résine. Les protéines retenues sur la colonne sont éluées successivement par des solutions de PBS Tween 20 0,02% ajustées respectivement à 0,65M NaCl, 2,15M NaCl, puis 6M urée. L'élution est réalisée par 250 ml de ces 3 solutions.

Chaque fraction est collectée. Les quantités de protéines et d'activité biologique sont évaluées.

Comme le montre la figure 3, qui représente le spectre obtenu en électrophorèse en condition réductrice sur gel de polyacrylamide, une protéine homogène, de poids moléculaire de 19000 daltons environ est obtenue par élution avec du tampon PBS-Tween 20 0,2%-NaCl 2M (ligne c sur cette figure 3, sur laquelle on a également fait figurer, en ligne a, le spectre de cette fraction F avant chromatographie sur la résine Sephadex carboxyméthylbenzylamide sulfonate(SCMBS), et, en ligne b, la fraction non retenue sur la résine précitée).

Le chromatogramme obtenu en HPLC phase inverse de 5 µg de la fraction F, ainsi purifiée sur Heparin Sepharose, fait l'objet de la figure 4. Il comporte trois bandes éluées entre 24 et 26% d'acétonitrile, la bande centrale majoritaire représentant environ 50-60% du matériel élué. (Conditions de cette chromatographie : Colonne $C_4$ taille des pores : 300A ; éluant : $H_2O/CH_3CN$/0,1% d'acide trifluoro-

acétique). La purification est très satisfaisante, les résultats, exprimés à partir de 1kg de matériel de départ, étant rapportés dans le Tableau ci-après .

Tableau

| Fractions | Quantité de protéines(mg) |
|---|---|
| Fraction acido-soluble | 175 |
| Fraction (1-10) (Heparin-Sepharose) | 0,020 |
| Chromatographie sur SCMBS.: | |
| -Fraction non retenue ( 0,65M NaCl) | 0,004 |
| -Fraction éluée ( 2M NaCl) | 0,016 |

Ib - MISE EN OEUVRE DU SECOND PROCEDE

On prépare un extrait acido-soluble de cerveau et on réalise la chromatographie d'affinité sur la résine I Heparin Sepharose, comme décrit aux points respectivement (a) et (b) de IA. On a analysé la fraction F obtenue en HPLC phase inverse. Le chromatogramme résultant fait l'objet de la figure 5. Il comporte trois bandes éluées entre 24 et 26% d'acétonitrile et fait apparaître une bande centrale majoritaire représentant environ 50 à 60% du matériel élué. (mêmes conditions de chromatographie que celles indiquées au paragraphe Ia - point (c) ; 56µg de la fraction (1-10) ).

## II - MISE EN EVIDENCE DE L'ACTIVITE STIMULATRICE DES FACTEURS DE CROISSANCE PRESENTEE PAR LA FRACTION PROTEIQUE SELON L'INVENTION

Le test biologique mis en oeuvre est basé sur la stimulation de cellules confluentes par des fractions contenant un facteur de croissance. L'évaluation de cette stimulation est faite par mesure de l'accroissement de la population cellulaire après incorporation d'un désoxynucléotide marqué, la thymidine tritiée qui est détectée par comptage de la radio-activité.

Les cellules sont ensemencées dans des puits d'une boîte de culture. Six jours plus tard, les cellules sont confluentes dans les puits tandis que le milieu est épuisé en sérum. C'est à ce stade que les facteurs de croissance peuvent être inoculés, afin de tester leur potentiel à stimuler les cellules dans les conditions optimales.

(a) Protocole expérimental (Déroulement du test)

Les facteurs de croissance h-aFGF ou h-bFGF avec ou sans la fraction protéique de l'invention, sont filtrés stérilement, sur filtre 0,22 µm (Millex) et sont inoculés en triplicate sous des volumes de 10 à 50 µl à 5 dilutions successives. 24 heures plus tard, on ajoute aux cultures 1µCi de thymidine tritiée (25 Ci/mM, CEA France), préalablement diluée avec du PBS stérile.

L'incorporation de thymidine tritiée est arrêtée après 4 h par aspiration du milieu de culture, suivie de deux rinçages avec du PBS. Les cellules sont ensuite lysées avec 500 µl de soude 0,1N. Chaque lysat est aspiré et dilué dans 3 ml de liquide scintillant (PACKARD INSTAGEL). La radioactivité incorporée est mesurée à l'aide d'un compteur à scintillation liquide (LKB RACKBETA). Chaque comptage est effectué pendant 60 secondes.

(b) Expression des résultats

Dans les conditions expérimentales choisies, il existe une relation entre la quantité de facteur de

croissance ajoutée au milieu et la quantité de radioactivité incorporée par les cellules en culture dans ce milieu. On peut tracer une dose - réponse en exprimant la radioactivité incorporée en fonction de la quantité de protéines ajoutées par millilitre de milieu de culture, l'axe des abcisses étant porté selon une échelle logarithmique.

Des expériences ont fait apparaître que la fraction protéique selon l'invention ne possède en elle-même aucune activité sur la croissance des cellules BEL, CC1-39 ou FR-3 T3.

En revanche, lorsqu'elle est ajoutée aux facteurs croissance h-aFGF et h-BFGF, à des concentrations du même ordre de grandeur (ng/ml), elle accroît par un coefficient de 2 à 5 l'activité stimulatrice des facteurs de croissance.

C'est ce que montrent les courbes dose-réponse des figures 6 à 8, les figures 6 et 7 illustrant l'effet potentialisateur de la fraction protéique selon l'invention sur la stimulation respectivement des fibroblastes FR-3T3 et CCL-39 par les facteurs h-aFGF (A) et h-bFGF (B) en l'absence de sérum de veau foetal, et la figure 8 illustrant l'effet potentialisateur de la fraction protéique selon l'invention sur la stimulation des cellules BEL par le facteur h-aFGF en présence d'héparine, et en absence de sérum de veau foetal (A) ou en présence de 6 % de sérum de veau foetal :

Figures 6 et 7 :

●——● sans la fraction protéique de l'invention

o——o avec la fraction protéique de l'invention (2 ng/ml dans le cas de la figure 6 et 20 ng/l dans le cas de la figure 7).

Figure 8 :

o——o h-aFGF seul

+——+ avec héparine 5 µg/ml

●——● avec héparine 5 µg/ml et fraction protéique de l'invention 13 ng/l.

L'examen de l'ensemble de ces figures 6 à 8 permet de constater que la fraction protéique de l'invention est capable de potentialiser l'effet stimulateur des facteurs h-FGFs sur les trois types cellulaires précités, en présence ou en l'absence de sérum de veau foetal.

La figure 8 montre également que la présence d'héparine aux concentrations utilisées est sans influence sur l'effet potentialisateur de la fraction protéique selon l'invention.

Il a également été démontré que la fraction protéique selon l'invention présente la même action sur les cellules bovines endothéliales de la cornée .

De plus, on a pu montrer que les sous-fractions correspondant à chacun des trois pics obtenus en HPLC présentaient la propriété de stimuler les facteurs de croissance. Leur utilisation, dans ce but, fait également l'objet de la présente invention .

III - OBTENTION DE LA FRACTION PROTEIQUE SELON L'INVENTION A PARTIR D'UN EXTRAIT PLACENTAIRE HUMAIN.

On a procédé comme au paragraphe II (a), (b), (c) pour l'extrait acido-soluble de cerveau, l'extrait placentaire humain ayant été dissous dans un tampon PBS 0,65M NaCl. Une protéine homogène, de poids moléculaire de 19 000 daltons, est obtenue par élution par NaCl 2M.

La figure 9 représente le spectre de cette fraction protéique F, ce spectre étant obtenu par électrophorèse en condition réductrice (ligne a) et en condition non réductrice (ligne b), sur un gel de polyacrylamide. L'observation de ce spectre montre une identité entre les deux fractions F.

Quant à la figure 10, elle représente une courbe dose-réponse analogue aux figures 6 à 8, illustrant l'effet potentialisateur de la fraction protéique selon l'invention sur la stimulation des fibroblastes CCL-39 par le facteur

h-aFGF en l'absence de sérum de veau foetal, le mode operatoire étant identique à celui indique au paragraphe II(a).

Légende de la Figure 10 :

•——• h-aFGF seul

⊙——⊙ avec la fraction protéique de l'invention (20 µl)

On constate que le facteur h-aFGF, à très faible concentration, présente peu d'activité. La solution de la fraction protéique de l'invention à 20 µl par ml de milieu n'a pas d'activité (BF - bruit de fond). Quand 20 µl par ml de milieu sont ajoutés à chaque fraction de h-aFGF, il y a une activation de l'incorporation de thymidine dans les cellules fibroblastes.

Comme dans le cas précédent, des résultats analogues ont été obtenus sur d'autres cellules, comme les cellules épithéliales du cristallin.

Les nouvelles fractions protéiques selon l'invention peuvent être utilisées dans toutes les applications des facteurs de croissance, comme co-facteurs stimulateurs ou potentialisateurs desdits facteurs, notamment pour la fabrication de milieux de culture cellulaire, la cicatrisation de la peau, la survie de cellules nerveuses, le contrôle de la vascularisation, ou encore comme co-facteur pour l'angiogénése ainsi que dans le domaine cosmétique, par application locale sur la peau ou les cheveux.

REVENDICATIONS

1 Procédé pour l'obtention d'une fraction protéique ayant une activité au moins partielle de co-facteur des facteurs de croissance, caractérisé par le fait qu'il consiste à purifier un facteur de croissance par chromatographie d'affinité sur une résine I et/ou II,

- ladite résine I étant à base de polysaccharides sur lesquels est fixée de l'héparine, et
- ladite résine II étant constituée d'un dextrane réticulé qui présente des fonctions carboxyméthyle,éventuellement des fonctions carboxyméthylbenzylamide et qui présente également des fonctions carboxyméthylbenzylamide sulfonate, et dont le comportement biologique est celui de l'héparine,

en réalisant, dans le cas de la chromatographie d'affinité sur la résine I, une élution avec un tampon pH neutre, d'une force ionique équivalente à celle d'une solution de NaCl voisine de 1 M, et, dans le cas de la chromatographie d'affinité sur la résine II, une élution avec un tampon pH neutre, d'une force ionique équivalente à celle d'une solution de NaCl voisine de 2 M.

2 - Procédé selon la revendication 1, caractérisé par le fait qu'on purifie le facteur de croissance par chromatographie d'affinité sur la résine I, afin d'obtenir une fraction F, constituée par des traces du facteur aFGF et d'une fraction protéique possédant la propriété de stimuler et potentialiser les facteurs de croissance, puis qu'on purifie ladite fraction F par chromatographie d'affinité sur la résine II, afin d'obtenir la fraction protéique précitée possédant la propriété de stimuler et potentialiser les facteurs de croissance.

3 - Procédé selon la revendication 1, caractérisé par le fait qu'on purifie le facteur de croissance par chromatographie d'affinité sur la résine I seulement, pour obtenir ladite fraction F, telle que définie à la revendication 2.

4 - Procédé selon la revendication 1, caractérisé par le fait qu'on purifie le facteur de croissance par chromatographie d'affinité sur la résine II.

5 - Procédé selon l'une des revendications 1 à 4, caractérisé par le fait qu'on utilise, comme matériel de départ, un facteur de croissance d'origine humaine ou animale.

6 - Procédé selon la revendication 5, caractérisé par le fait qu'on utilise, comme matériel de départ, un facteur de croissance issu du cerveau humain.

7 - Procédé selon la revendication 6, caractérisé par le fait qu'on utilise, comme matériel de départ, un extrait acido-soluble de cerveau humain.

8 - Procédé selon la revendication 5, caractérisé par le fait qu'on utilise, comme matériel de départ, un facteur de croissance issu du placenta humain.

9 - Procédé selon la revendication 8, caractérisé par le fait qu'on utilise,comme matériel de départ, un extrait placentaire humain.

10 - Procédé selon la revendication 7, caractérisé par le fait que l'on obtient l'extrait acido-soluble par la succession d'étapes suivantes :

- broyage respectivement du cerveau humain en tampon phosphate à pH neutre ;
- obtention des protéines par précipitation au sulfate d'ammonium entre 20 et 60% :
- isolement des protéines restant solubles en milieu acide, au voisinage de pH 3 ; et
- dialyse contre un tampon à pH neutre.

11 - Procédé selon l'une des revendications 1 à 10, caractérisé par le fait qu'on utilise, comme résine I, une résine "Heparin Sepharose"

12 - Procédé selon l'une des revendications 1 à 11, caractérisé par le fait que l'on utilise, comme résine II, une résine "Sephadex carboxyméthylbenzylamide sulfonate", qui possède une activité "heparin-like" de

0,2UT/mg et dont la structure est un polymère constitué par les motifs suivants :

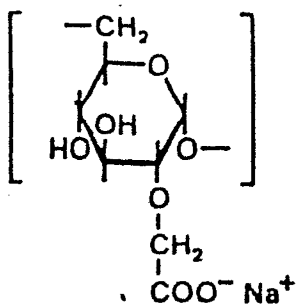

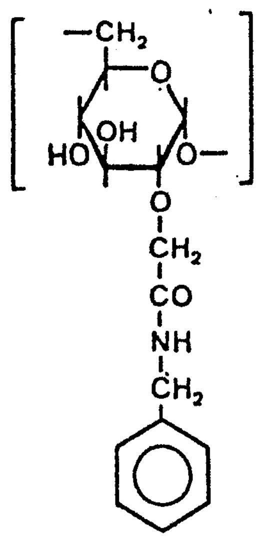

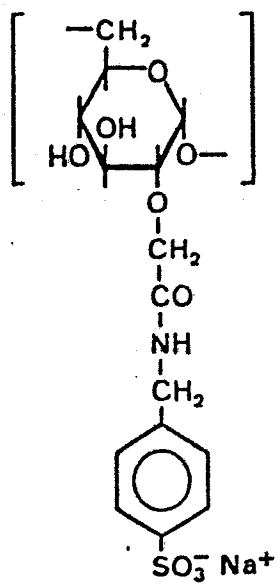

$[COO^-] = 81\%$

$[SO_3^-] = 9\%$

13 - Procédé selon l'une des revendications 1 à 12, caractérisé par le fait que l'on sépare en sous-fractions la fraction protéique obtenue par la technique de HPLC, lesdites sous-fractions ayant également l'activité de co-facteur des facteurs de croissance.

14 - Fractions protéiques obtenues par le procédé tel que défini à l'une des revendications 1 à 13.

15 - Fraction protéique, caractérisée par le fait que son poids moléculaire est déterminé comme étant de 20000 daltons environ en électrophorèse en condition non réductrice, laquelle fournit un spectre à bande unique, et comme étant de 19000 daltons environ en électrophorèse en condition réductrice, laquelle fournit un spectre à deux bandes, et que, par la méthode de chromatographie en phase

liquide haute performance, elle fournit un spectre à trois pics.

16 - Fraction protéique selon la revendication 15, caractérisée par le fait que sa composition en acides aminés déterminée après hydrolyse acide et avant HPLC phase inverse (calculée par rapport à un poids moléculaire de 19000 daltons) est la suivante (acide aminé/nombre de résidus de l'acide aminé par molécule) :
Asx/12 ; Glx/18 ; Ser/15 ; His/15 ; Gly/23 ; Thr/11 ; Arg/15; Ala/14 ; Tyr/4 ; Met/3 ; Val/6 ; Phe/6 ; Ile/4 ; Leu/9 ; Lys/26.

17 - Fraction protéique selon l'une des revendications 14 à 16, caractérisée par le fait qu'elle possède la propriété de stimuler les facteurs de croissance.

18 - Fraction protéique selon l'une des revendications 15 à 17, caractérisée par le fait qu'elle est obtenue par le procédé tel que défini à la revendication 2.

19 - Chacune des trois sous-fractions résultant d'une séparation par la technique de HPLC de la fraction protéique telle que définie à l'une des revendications 15 à 18.

20 - Utilisation des fractions protéiques telles que définies à l'une des revendications 14 à 18, et des trois sous-fractions telles que définies à la revendication 19, dans toutes les applications des facteurs de croissance, comme co-facteurs, agents stimulateurs ou potentialisateurs desdits facteurs, notamment pour la fabrication de milieux de culture cellulaire, la cicatrisation de la peau, la survie de cellules nerveuses, le contrôle de la vascularisation, ou encore comme cofacteur pour l'angiogénése ainsi que dans le domaine cosmétique, par application locale sur la peau ou les cheveux.

FONDÉ EN 1819

CABINET HARLÉ & PHÉLIP

CONSEILS EN PROPRIÉTÉ INDUSTRIELLE EUROPEAN PATENT ATTORNEYS
MEMBRES DE L'ASSOCIATION DES CONSEILS EN PROPRIÉTÉ INDUSTRIELLE
MEMBRES DE LA CHAMBRE DES SPÉCIALISTES EN MARQUES ET MODÈLES
21, RUE DE LA ROCHEFOUCAULD 75009 PARIS

TÉL. (1) 42.80.68.99 TELEX 640251 F CABLES BREVMARQ-PARIS

TELEFAX (II + III) - (1) 48.74.51.37

**0254616**

Notre réf : 9938 EU 460
PH/CH/DO

EUROPEAN PATENT OFFICE
P.B. 5818 patentlaan 2
2280 HV RIJSWIJK (ZH)
PAYS-BAS

Paris, le 23 juillet 1987

Messieurs,

Demande de brevet européen n° 87-401 509.2 déposée le 30 juin 1987 au nom du CENTRE NATIONAL DE LA RECHERCHE SCIENTIFIQUE pour : "Procédé d'obtention de nouvelles fractions protéiques à partir des activités facteurs de croissance, ces fractions et leur application comme agents de stimulation et de potentialisation des activités facteurs de croissance". Invention : ULHRICH et al.

---

Nous souhaiterions corriger une erreur matérielle qui s'est glissée à la page 13, ligne 21 de la description telle que déposée.

En effet, à cette ligne, «II» a été mis pour «Ia».

Ce paragraphe III concernant l'obtention d'une fraction protéique selon l'invention, non plus à partir d'un extrait acido-soluble de cerveau comme au paragraphe I, mais un extrait placentaire humain, on ne peut se reporter qu'au paragraphe I, et non au paragraphe II, qui porte sur une mise en évidence de l'activité stimulatrice sur les facteurs de croissance présentés par la fraction protéique de l'invention.

Par ailleurs, on a utilisé, dans le cas de l'extrait placentaire humain, le premier procédé du point I (et non pas le deuxième), ce premier procédé comportant effectivement les trois étapes (a), (b) et (c).

Nous joignons à la présente, en triple exemplaire, une nouvelle page 13 dûment corrigée, et il nous serait agréable que vous puissiez substituer cette page à l'ancienne.

Vous en remerciant à l'avance, nous vous prions d'agréer, Messieurs, l'expression de nos sentiments les meilleurs.

B. PHELIP G. TANGUY

P.J. - nouvelle page 13 en 3 exemplaires

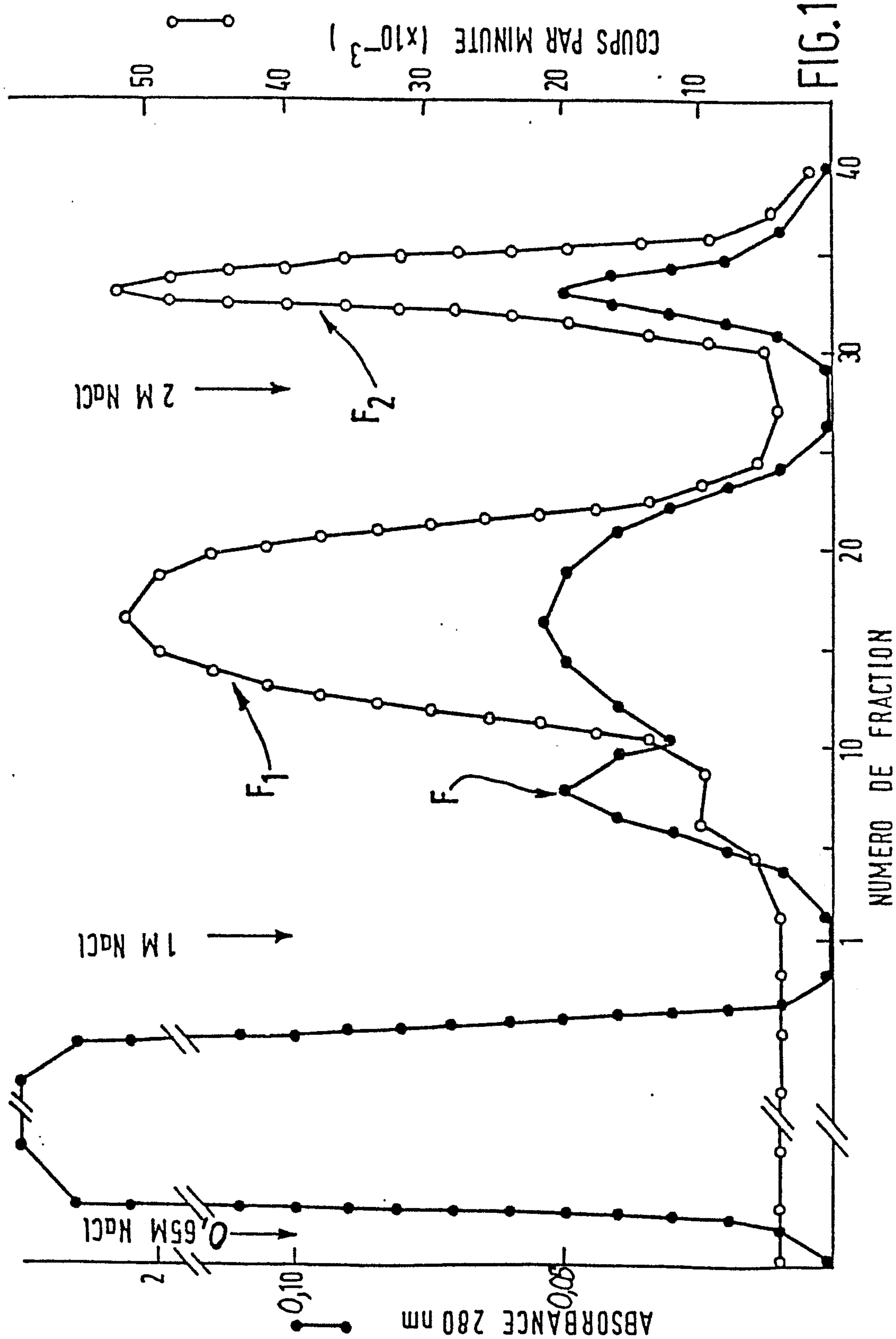
COUPS PAR MINUTE (x10-3)
50
40
30
20
10
2 M NaCl
1 M NaCl
0,65M NaCl
F2
F1
F
40
30
20
10
1
NUMERO DE FRACTION
2
0,10
0,05
ABSORBANCE 280 nm

FIG.1

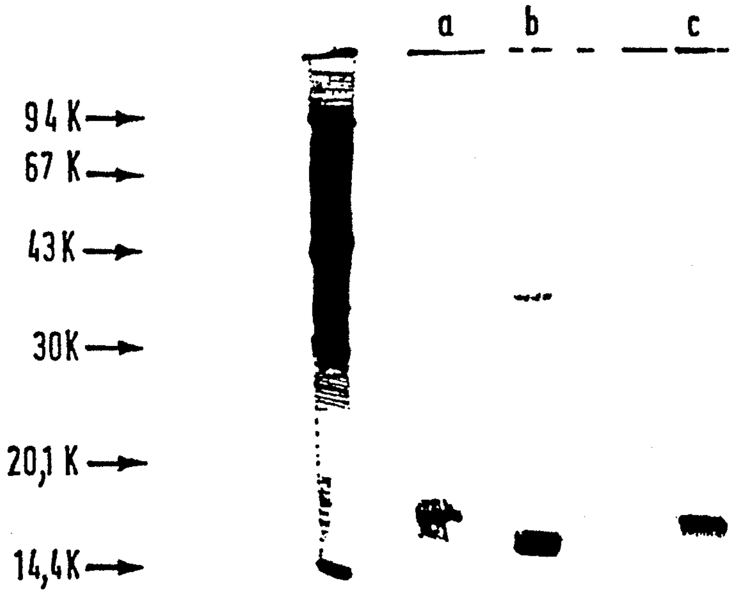


FIG.2

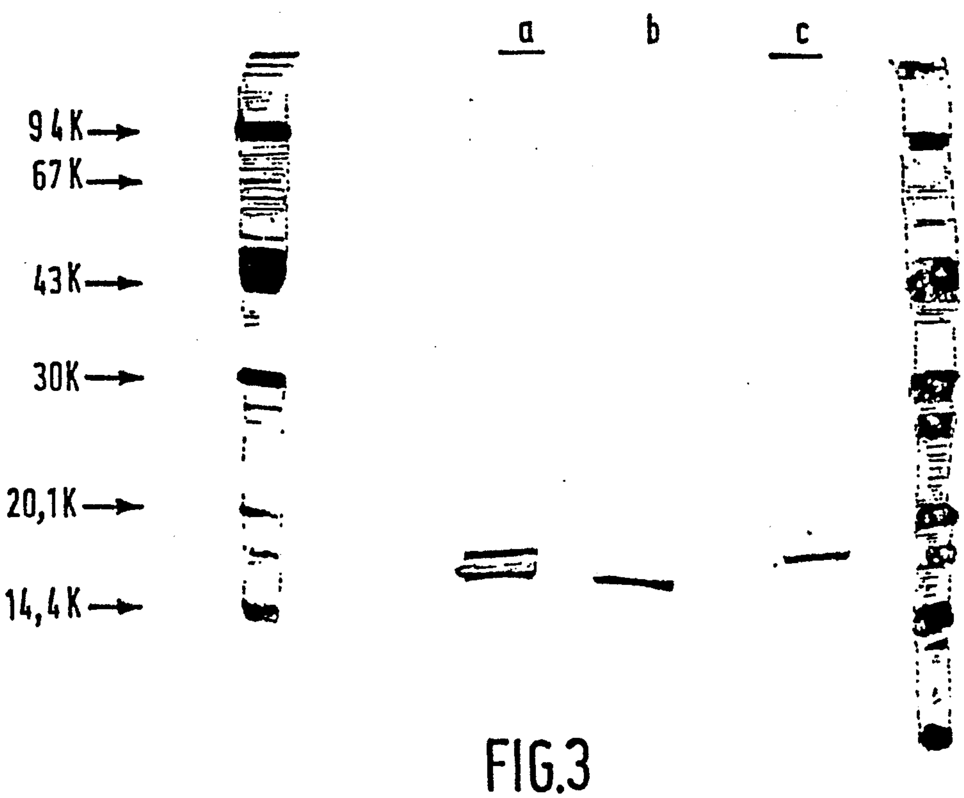


FIG.3

FIG. 4

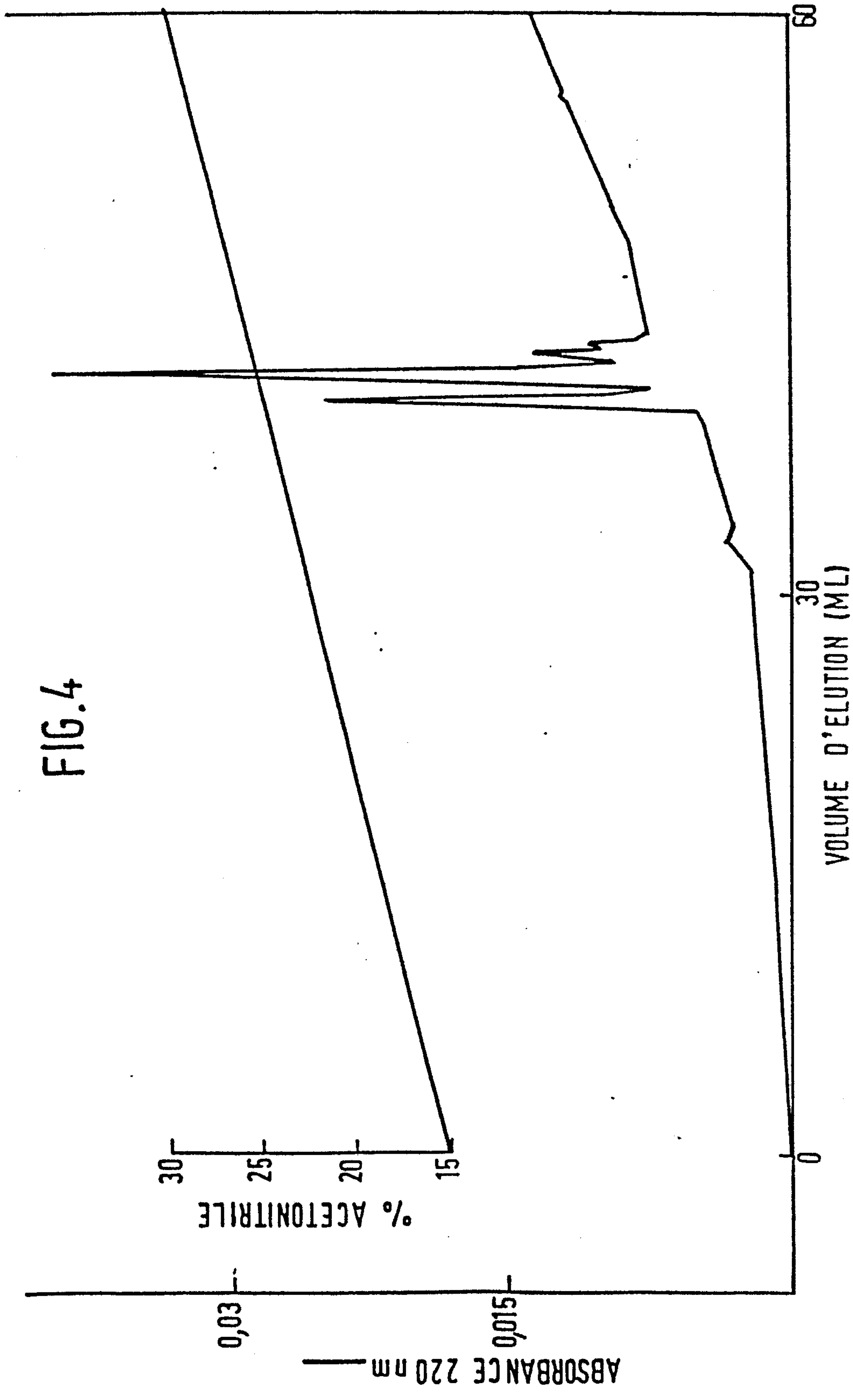
% ACETONITRILE
15
20
25
30
ABSORBANCE 220 nm
0,015
0,03
VOLUME D'ELUTION (ML)
0
30
60

0254616

9938EXT



FIG.5

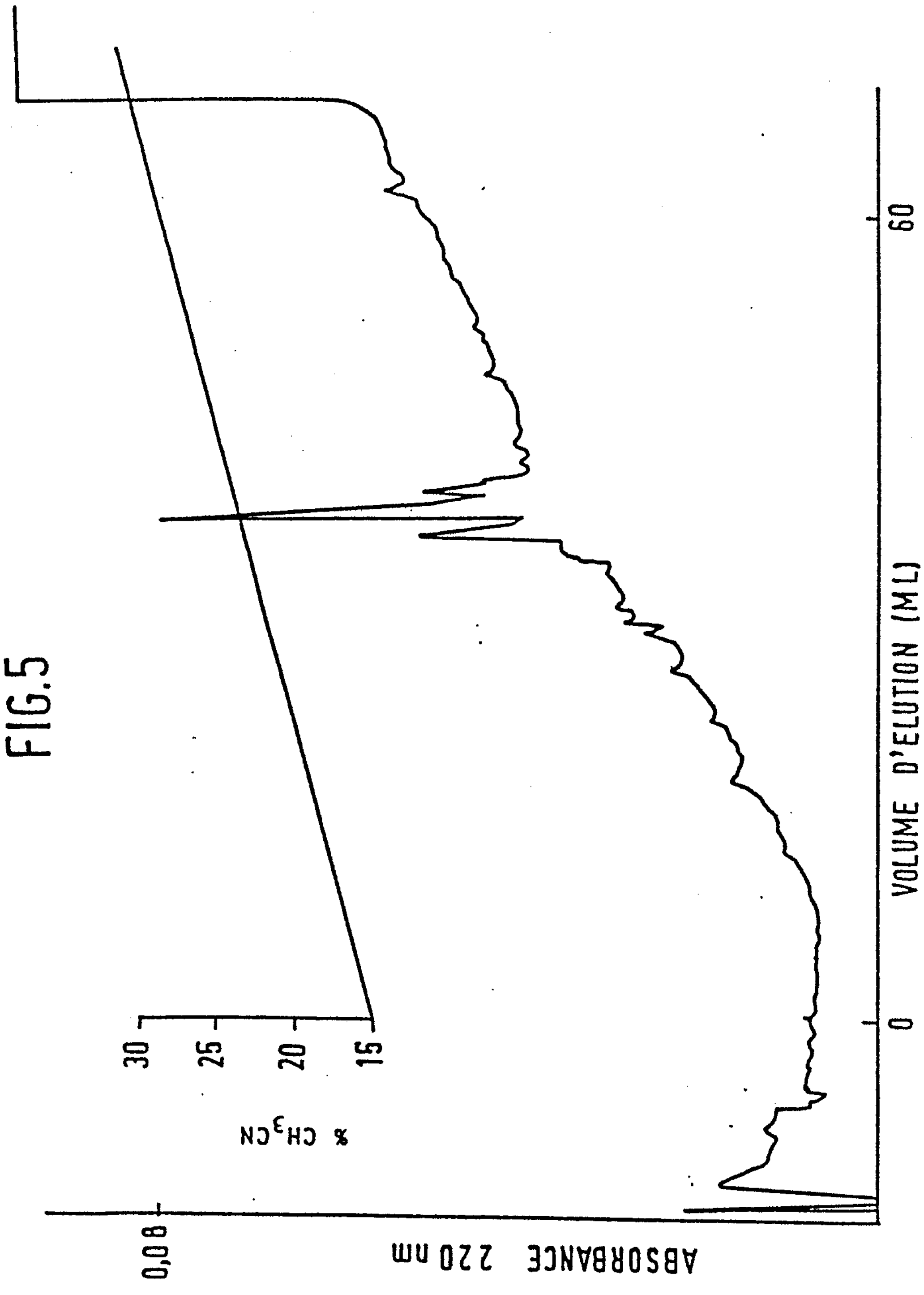

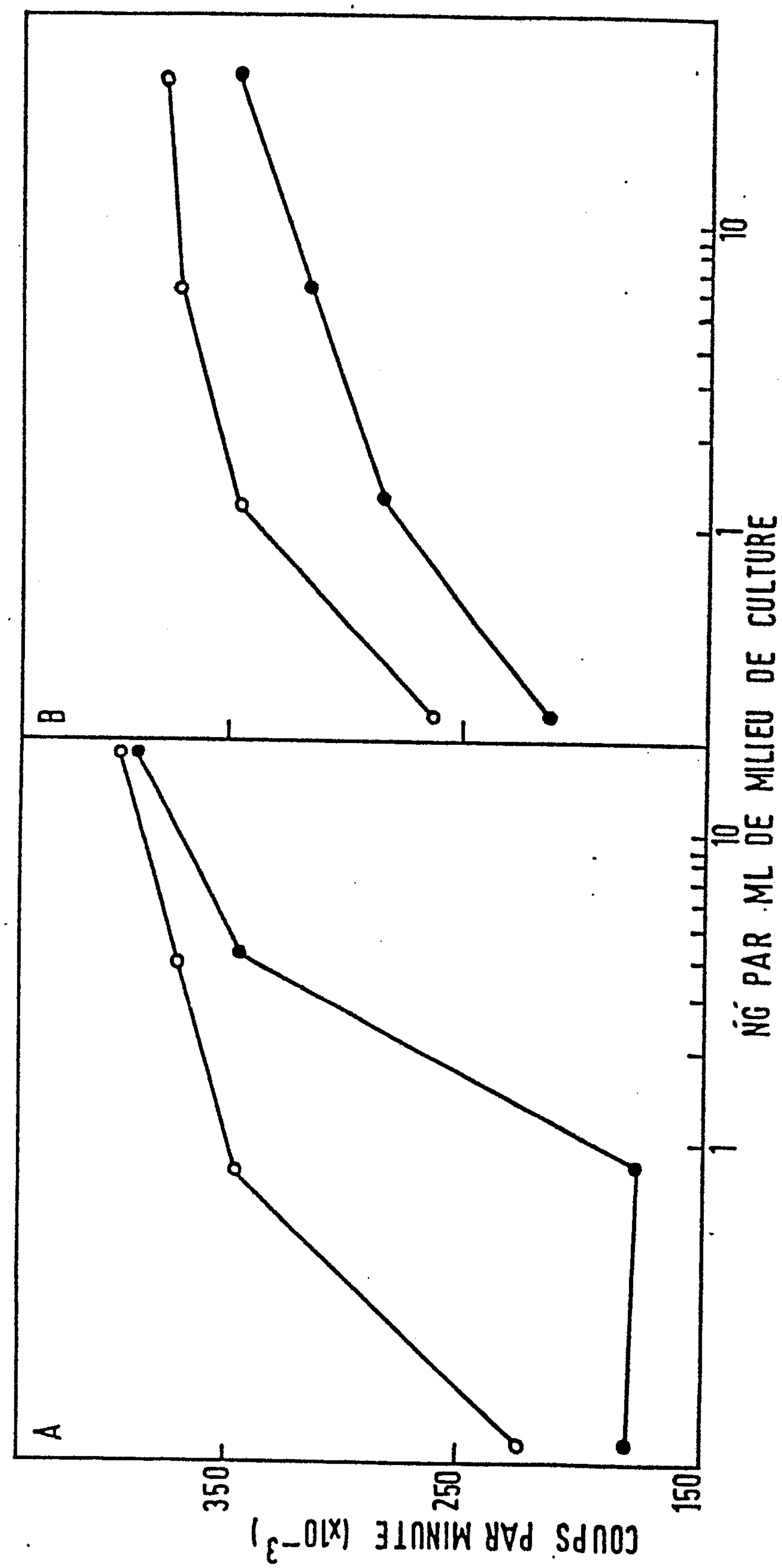
A
B
COUPS PAR MINUTE (x10⁻³)
350
250
150
1
10
1
10
NG PAR ML DE MILIEU DE CULTURE

FIG.6

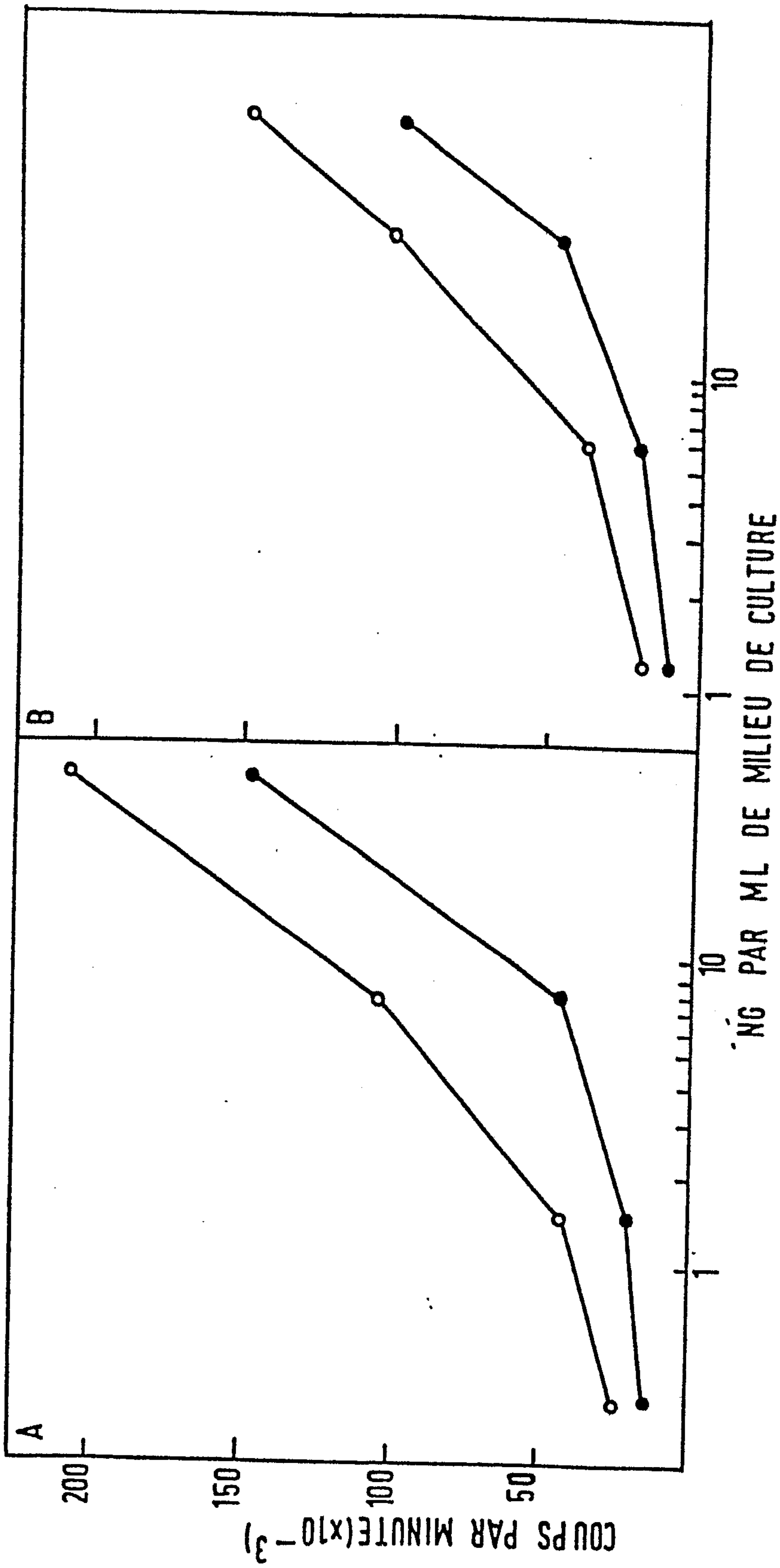
A
B
200
150
100
50
1
10
COUPS PAR MINUTE(x10 − 3)
NG PAR ML DE MILIEU DE CULTURE

FIG.7

9938EXT



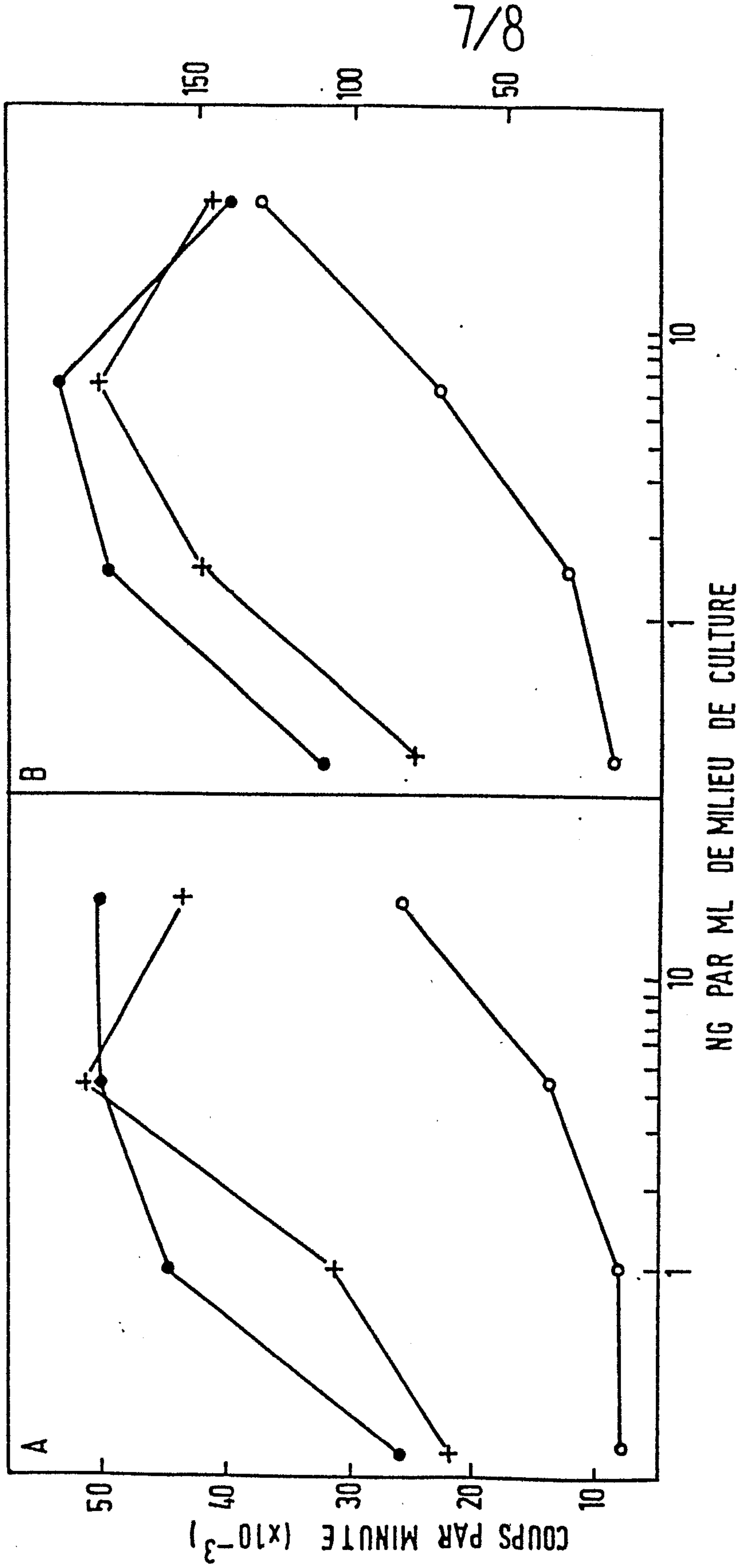


FIG.8

FIG.10

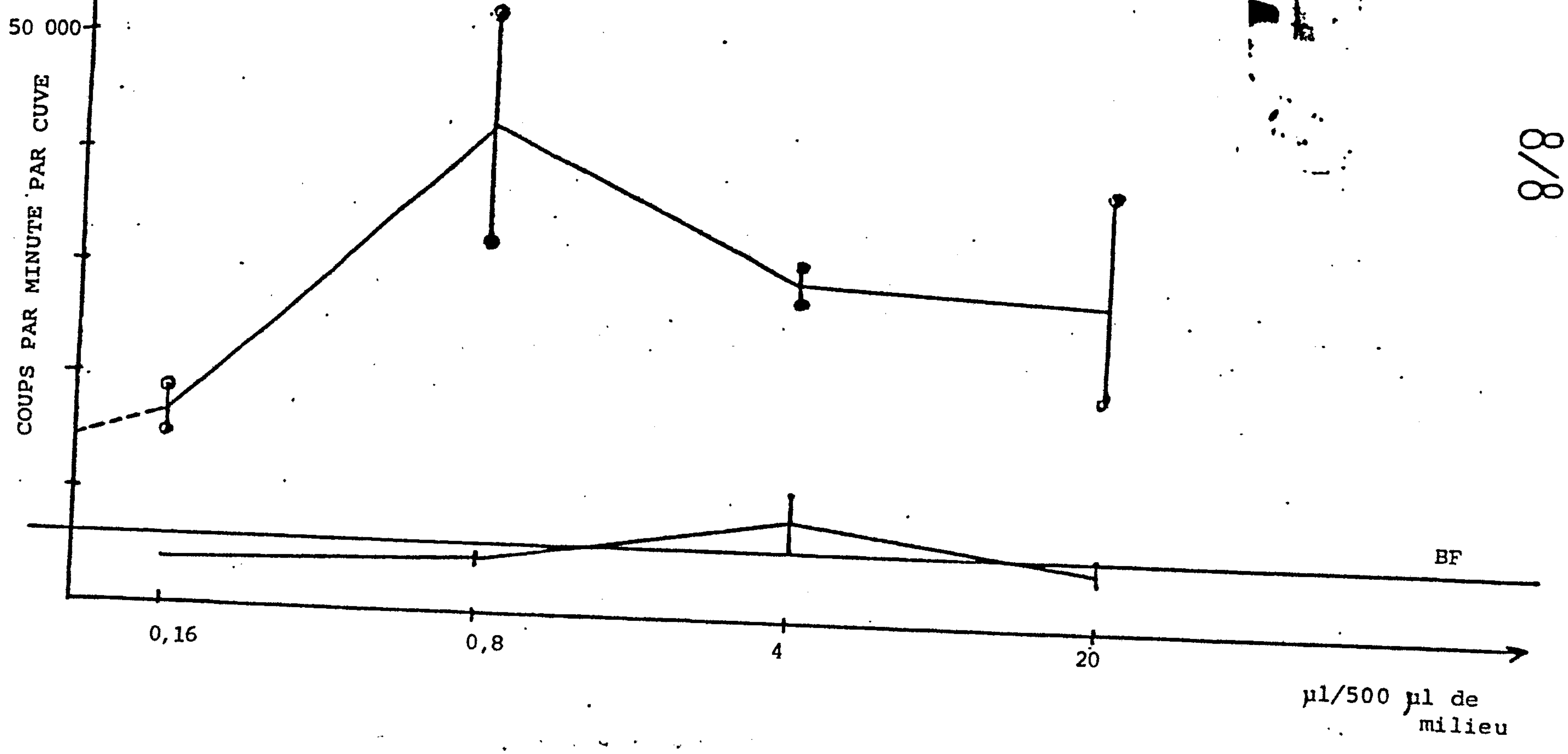
50 000
COUPS PAR MINUTE PAR CUVE
BF
0,16
0,8
4
20
µl/500 µl de milieu

FIG.9

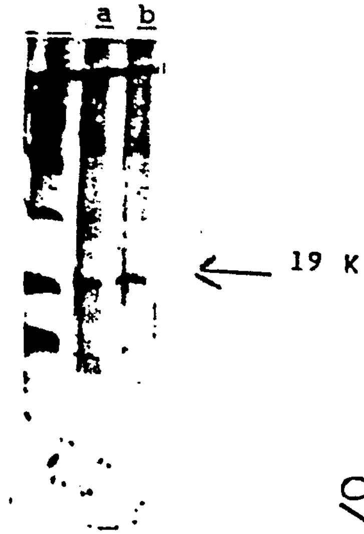
a b
19 K

0254616

Office européen des brevets

# RAPPORT DE RECHERCHE EUROPEENNE

Numero de la demande

EP 87 40 1509

**DOCUMENTS CONSIDERES COMME PERTINENTS**

| Catégorie | Citation du document avec indication, en cas de besoin, des parties pertinentes | Revendication concernée | CLASSEMENT DE LA DEMANDE (Int Cl 4) |
|---|---|---|---|
| A | BIOCHEMISTRY, vol. 23, no. 26, 18 décembre 1984, pages 6295-6299, American Chemical Society; R.R. LOBB et al.: "Purification of two distinct growth factors from bovine neural tissue by heparin affinity chromatography" * Page 6296, colonne 2, lignes 32-40; figure 1, pic H2A; page 6297, colonne 1, lignes 1-30; page 6297, colonne 2, lignes 1-16 * | 1-3 | C 07 K 15/00 // A 61 K 35/30 A 61 K 35/50 A 61 K 37/02 |
| A | PROC. NATL. ACAD. SCI. USA, vol. 82, février 1985, pages 805-809; M. KLAGSBRUN et al.: "Heparin affinity of anionic and cationic capillary endothelial cell growth factors: analysis of hypothalamus-derived growth factors and fibroblast growth factors" * Page 807, colonne 2, lignes 11-24; page 808, colonne 1, lignes 1-4; figure 6 * | 1-3 | DOMAINES TECHNIQUES RECHERCHES (Int Cl 4) A 61 K |
| D,A | ANALYTICAL BIOCHEMISTRY, vol. 154, 1986, pages 1-14, Academic Press, Inc.; R.R. LOBB et al.: "Review: purification of heparin-binding growth factors" | | |

--- -/-

Le présent rapport de recherche a été établi pour toutes les revendications

| Lieu de la recherche | Date d'achèvement de la recherche | Examinateur |
|---|---|---|
| LA HAYE | 24-09-1987 | SKELLY J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

Office européen des brevets

# RAPPORT DE RECHERCHE EUROPEENNE

Numéro de la demande
EP 87 40 1509



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | BIOCHEMISTRY, vol. 24, no. 19, septembre 1985, pages 4969-4973, American Chemical Society, Salem, Massachusetts, US; R.R. LOBB et al.: "Induction of angiogenesis by bovine brain derived class 1 heparin-binding growth factor"<br>* En entier *<br>----- | 20 | |

DOMAINES TECHNIQUES RECHERCHES (Int Cl 4)

Le present rapport de recherche a été établi pour toutes les revendications

| Lieu de la recherche | Date d'achèvement de la recherche | Examinateur |
|---|---|---|
| LA HAYE | 24-09-1987 | SKELLY J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82